# EUROPEAN PATENT APPLICATION

(11) **EP 1 679 088 A2**
(43) Date of publication of application: **12.07.2006**
(21) Application number: 06000308.4
(22) Date of filing: 09.01.2006
(51) Int. Cl.: A61L 27/32, A61F 2/30, A61F 2/32, A61F 2/38, A61F 2/40

(54) **Medical devices coated with a nanostructured material**

(30) Priority: 07.01.2005 US 642449 P
(71) Applicant: Inframat Corporation, Farmington, CT 06032 (US)
(72) Inventor: Zhang, Zongtao, Unionville, CT 06085 (US); Ma, Xinqing, Willington, CT 06279 (US); Roth, Jeffrey David, Coventry, CT 06238 (US); Xiao, T. Danny, Willington, CT 06279 (US); Krajewski, Jay Alexander, Coventry, CT 06238 (US)
(74) Representative: Maiwald Patentanwalts GmbH

(57) **Abstract**

A medical device generally includes a structural member having a surface wherein at least a portion of the surface is coated with a nanostructured material to a thickness of at least about 25 micrometers, and wherein the nanostructured material comprises a ceramic, ceramic composite, ceramic metal composite, or a combination comprising at least one of the foregoing. The implants have increased service lifetimes owing in part to improved wear and abrasion resistance, and may be useful for partial or full replacement of articulating and flexible hinge joints.

## Description

### CROSS REFERENCE TO RELATED APPLICATION

This application claims the benefit of United States Provisional Patent Application Serial Number 60/642,449 filed January 07, 2005, which is incorporated herein by reference in its entirety.

### STATEMENT REGARDING FEDERALLY SPONSORED RESEARCH

The United States Government has certain rights in this invention pursuant to National Science Foundation Grant Number DMI-0319325.

### BACKGROUND

The present invention generally relates to coatings and more specifically to coatings deposited onto surfaces of medical devices.

Surgical implantation of medical devices can structurally compensate for diseased, damaged, or missing skeletal anatomical elements, such as articulating bone joints and related bone structures. Although some devices can last a few decades, a significant number fail within 10 to 15 years. While this may be acceptable for some older patients, longer service lives are needed as younger patients and more active older patients increasingly undergo replacement surgery.

The service life of a medical device is largely dependent on the amount of wear and tear to which its load bearing surfaces are exposed. Localized stress from interaction between these surfaces generates small particulate debris that breaks off and contaminates the synovial fluid surrounding the implant. In response, the body's immune system will secrete enzymes in an attempt to degrade these particles. However, these enzymes often kill adjacent bone cells or cause osteolysis resulting in mechanical loosening and failure of the implant. Further, protuberances in a load bearing surface can scratch an opposing load bearing surface, which leads to microcrack formation and ultimately to catastrophic fracture of the implant. Therefore, it is important to minimize the wear rate of implants' load bearing surfaces.

It is an object of the invention to provide implant devices with improved surfaces. It would be particularly advantageous if these devices could minimize release of wear debris or prevent microcracks from starting and/or increasing in size to an extent effective to prolong service life of the implant.

### BRIEF SUMMARY

A medical device includes a structural member having a surface wherein at least a portion of the surface is coated with a nanostructured material to a thickness of at least about 25 micrometers, and wherein the nanostructured material comprises a ceramic, ceramic composite, ceramic metal composite, or a combination comprising at least one of the foregoing.

In another embodiment, the medical device includes a first structural member having a first surface and a second structural member having a second surface, wherein at least a portion of one or both of the first and second surfaces is coated with a nanostructured material to a thickness of at least about 25 micrometers.

A method includes surgically implanting the medical device comprising a structural member having a surface, wherein at least a portion of the surface is coated with a nanostructured material to a thickness of at least about 25 micrometers, and wherein the nanostructured material comprises a ceramic, ceramic composite, ceramic metal composite, or a combination comprising at least one of the foregoing.

A method of making a medical device includes coating at least a portion of at least one surface of the medical device with a film of a nanostructured material having a thickness of at least about 25 micrometers.

The above described and other features are exemplified by the following figures and detailed description.

### BRIEF DESCRIPTION OF THE DRAWINGS

Referring now to the figures, which are exemplary embodiments and wherein like elements are numbered alike:

Figure 1 depicts an artificial hip joint implant;

Figure 2 depicts an artificial knee joint implant;

Figure 3 is a scanning electron micrograph illustrating the thickness of a chromia coating deposited on a load bearing surface of a femoral head;

Figure 4 is a scanning electron micrograph illustrating the surface microstructure of a chromia coating deposited on a load bearing surface of a femoral head; and

Figure 5 is a transmission electron micrograph illustrating the grain size of a chromia coating deposited on a load-bearing surface of a femoral head.

### DETAILED DESCRIPTION

Medical devices and methods of making and using the devices with increased service life are described. The medical devices generally include devices that can be surgically implanted. Such implants generally include a structural member having a surface, which is at least partially coated with a thick film of a low friction, wear resistant, nanostructured material. Nanostructured materials can have superior properties compared to those with larger grain sizes including improved abrasion resistance and wear resistance. The improved abrasion resistance has been attributed to the increased hardness and ultrafine grain size of the nanostructured material. The ultrafine grain size is thought to alter the fracture and material removal mechanisms.

As used herein, the terms "first", "second", and the like do not denote any order or importance, but rather are used to distinguish one element from another, and the terms "the", "a", and "an" do not denote a limitation of quantity, but rather denote the presence of at least one of the referenced item. Furthermore, all ranges disclosed herein are inclusive of the endpoints and independently combinable.

The medical device may comprise a portion (i.e., for partial replacement) of an articulating or flexible joint. Alternatively, the medical devices may be a complete (i.e., for full replacement) articulating or flexible joint. Examples of articulating joints include those used in partial or full replacement of a joint in a knee, hip, elbow, ankle, spine, shoulder, wrist, or the like. Examples of flexible hinge joints include silastic and metacarpal-phalangeal joints used in partial or full replacement of a joint in a finger, toe, or the like.

In embodiments where the medical device is used for full replacement of a joint, the medical device generally comprises the structural member (hereinafter "first structural member") having the surface (hereinafter "first surface") and a second structural member having a second surface, wherein at least a portion of one or both of the surfaces are coated with the thick film of the low friction, wear resistant, nanostructured material. Depending on the particular type of medical device, the first and second surfaces can be pivotably engageable.

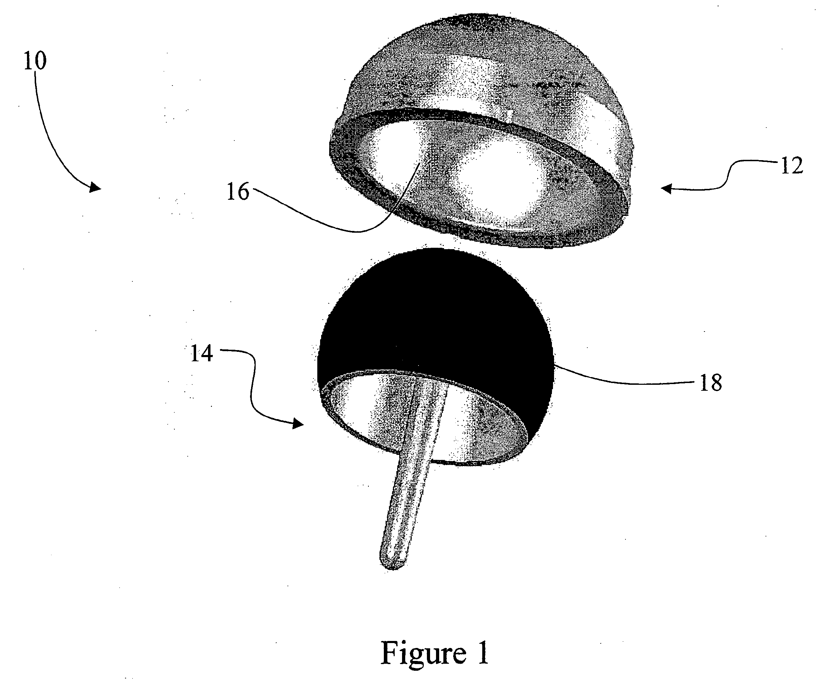

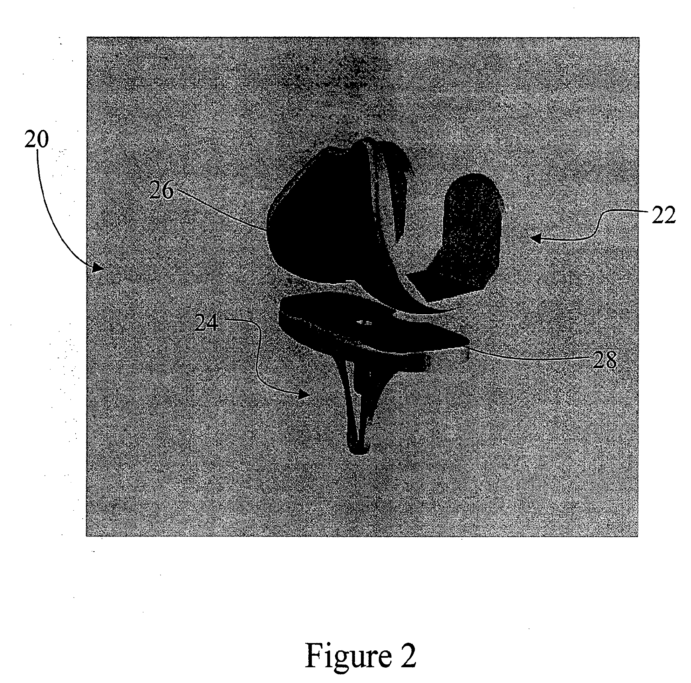

For illustrative convenience, Figures 1 and 2 show exemplary artificial hip and knee joint implants, respectively. The hip joint implant 10 includes as the first structural member a femoral head 14, and as the second structural member an acetabular socket 12 with a concave opening 16 as the second surface. In this illustration, the coating 18 is disposed on only the first surface. Analogously, the knee joint implant 20 includes as the first structural member a femoral condyle 22 and as the second structural member a tibia tray 24. In this embodiment, there are nanostructured thick film coatings 26 and 28 on the surfaces of both structural members. In an exemplary embodiment, the first and second surfaces are load-bearing surfaces, as is the case for hip joint implant 10 and knee joint implant 20.

Materials that can be used to form the first and/or second structural members include alloys, ceramics, polymers, and cartilage. The relatively corrosive environment combined with the low tolerance of the body for even minute concentrations of many metallic corrosion products eliminates from discussion many metals. Of the metallic candidates that have the required mechanical strength and biocompatibility, stainless steel alloys such as 316 L, chromium-cobalt-molybdenum alloys, titanium alloys such as Ti₆Al₄V, zirconium alloys, and combinations comprising at least one of the foregoing have proven suitable for use as structural members. Specific ceramics include alumina (Al₂O₃), zirconia (ZrO₂), chromia (Cr₂O₃), and the like. Specific polymers include ultra high molecular weight polyethylene (UHMWPE), highly cross-linked polyethylene (XLPE), and the like. Fiber- and/or particle-reinforced polymers may also be used.

If the medical device comprises two structural members, it is not critical that the first and second structural members be formed from the same type of material. However, when depositing the thick film, it is desirable that the nanostructured material be coated onto an alloy structural member. Thus, in exemplary embodiments, the first structural member is an alloy structural member such that the nanostructured material can be coated onto a surface of the alloy. It is also possible for both of the surfaces to be coated with a thick film of the nanostructured material as evidenced in Figure 2.

In one embodiment, the nanostructured material, which is coated onto at least a portion of one or both of the surfaces, is a ceramic material. Suitable ceramic compositions include hard phase metal oxides such as Al₂O₃, Cr₂O₃, ZrO₂, and the like; metal carbides such as Cr₃C₂, WC, TiC, ZrC, B₄C, and the like; diamond; metal nitrides such as cubic BN, TiN, ZrN, HfN, Si₃N₄, AlN, and the like; metal borides such as TiB₂, ZrB₂, LaB, LaB₆, W₂B₂, and the like; and combinations comprising at least one of the foregoing compositions. The wear characteristics of hard phase metal oxides, carbides, nitrides, and borides are superior to biomimetic materials such as hydroxyapatite and other phosphate-based materials, and are therefore preferred. In another embodiment, the nanostructured material is a ceramic composite comprising at least 51 volume percent (vol %), based on the total volume of the composite, of the aforedescribed suitable ceramic compositions and a binder phase of a relatively soft and low melting composition. Suitable ceramic binder phase compositions for the ceramic composite include SiO₂, CeO₂, Y₂O₃, TiO₂, and combinations comprising at least one of the foregoing ceramic binder phase compositions. In another embodiment, the nanostructured material is a ceramic metal composite (cermet). Suitable cermets include WC/Co, TiC/Ni, TiC/Fe, Ni(Cr)/Cr₃C₂, WC/CoCr, and combinations comprising at least one of the foregoing. The cermet may further include grain growth inhibitors such as TiC, VC, TaC, and HfC, or other additives such as Cr, Ni, B, and BN. In still another embodiment, the nanostructured material is a combination comprising at least one of the foregoing ceramics, ceramic composites, or cermets.

An average longest grain size dimension of the nanostructured material is about 1 nanometer (nm) to about 1000 nm. In one embodiment, the average longest grain size dimension of the nanostructured material is less than or equal to about 500 nm. In another embodiment, the average longest grain size dimension of the nanostructured material is less than or equal to about 400 nm. In another embodiment, the average longest grain size dimension of the nanostructured material is less than or equal to about 250 nm. In yet another embodiment, the average longest grain size dimension of the nanostructured material is less than or equal to about 200 nm. In yet another embodiment, the average longest grain size dimension of the nanostructured material is less than or equal to about 100 nm. In still another embodiment, the average longest grain size dimension of the nanostructured material is greater than or equal to about 10 nm. In still another embodiment, the average longest grain size dimension of the nanostructured material is greater than or equal to about 25 nm.

The thick film of the nanostructured material may be coated onto the surface by any known deposition method such as thermal spray, chemical vapor deposition, physical vapor deposition, sputtering, ion plating, cathodic arc deposition, atomic layer epitaxy, molecular beam epitaxy, powder sintering, electrophoresis, electroplating, injection molding, or the like. In an exemplary embodiment, thermal spray techniques including, for example, thermal spray using a powdered feedstock and solution precursor plasma spray, are used. Thermal spray techniques include coating processes in which a coating is applied to a substrate by deposition of materials in a molten or semi-molten state. Thermal spray may be performed with a detonation gun, a plasma gun, or a high velocity oxygen fuel (HVOF) gun. For ceramic and ceramic composite coatings, plasma thermal spray is more favorable, while HVOF is more favorable for cermet-containing film deposition.

In the HVOF spray process, nanometer-sized particles are desirably used as starting materials for reconstitution of a sprayable feedstock via a spray dry process. The substrate may optionally be prepared by degreasing and coarsening by sand blasting. As used herein, the term "substrate" refers to the surface of the structural member of the implant that will be coated with the thick film of the nanostructured material. A high velocity flame is generated by combustion of a mixture of fuel (e.g., propylene) and oxygen. The enthalpy and temperature can be adjusted by using different fuels, different fuel-to-oxygen ratios, and/or different total fuel/oxygen flow rates. The nature of the flame may be adjusted according to the ratio of fuel to oxygen. Thus, an oxygen-rich, neutral or fuel-rich flame can be produced. The feedstock is fed into the flame at a controlled feed rate via, for example, a co-axial powder port, melted and impacted on the target substrate to form a deposit/film. The film thickness may be controlled by the number of coating passes. The resultant films are optionally heat treated with an annealing step.

In the plasma spray process, nanometer-sized particles may be used as starting materials for the reconstitution of a sprayable feedstock via a spray dry process. The substrate may optionally be prepared by degreasing and coarsening by sand blasting. A plasma arc is a source of heat that ionizes a gas, which melts the coating materials and propels it to the work piece. Suitable gases include argon, nitrogen, hydrogen, and the like. Plasma settings, which may be varied, include current, voltage, working gases and their flow rates. Other process parameters include standoff distance, powder feed rate, and gun movements. Optimal conditions may be identified for each of the parameters without undue experimentation by one ordinarily skilled in the art. Film thickness may be controlled based on the number of coating passes. The resultant films are optionally heat treated with an annealing step.

Feedstock preparation for thermal spray techniques including HVOF and plasma spray may involve the formation of micrometer-sized agglomerates containing individual nanoparticles and an insulating material. The agglomerates are preferably substantially spherical, micron-sized granules containing agglomerated nanoparticles. Individual nanoparticles cannot be readily thermally sprayed directly owing to their fine size and low mass. Agglomeration of the nanoparticles to form micrometer-sized granules allows for formation of a suitable feedstock. Formation of the feedstock may comprise dispersion (e.g., by ultrasound) of the nanoparticles into a liquid medium; addition of a binder to form a solution; spray drying of the solution into agglomerated particles; and heating the agglomerated particles to remove organic binders and to promote powder densification.

In organic-based liquid media, the binder may comprise about 5% to about 15% by weight, and preferably about 10% by weight, of paraffin dissolved in a suitable organic solvent. Suitable organic solvents include, for example, hexane, pentane, toluene and the like, and combinations comprising one or more of the foregoing solvents. In aqueous liquid media, the binder may comprise an emulsion of polyvinyl alcohol (PVA), polyvinylpyrrolidone (PVP), carboxymethyl cellulose (CMC), another water soluble polymer, or a combination comprising one or more of the foregoing polymers, formed in de-ionized water. The binder may be present in an amount of about 0.5% to about 5% by weight of the total solution, and preferably from about 1% to about 10% by weight of the total aqueous solution. In one embodiment, the binder is CMC.

Thick films up to several hundred micrometers and even several millimeters thick may be produced in the solution precursor plasma spray process. The solution precursors may be fed into a plasma torch to deposit the thick film.

The precursor plasma spray process is described in more detail in commonly assigned U.S. Patent No. 6,447,848, incorporated herein by reference in its entirety. In the thermal spray process of forming the thick films from precursor solutions, three steps may be specifically involved: (1) preparing the precursor solution; (2) delivering the precursor solution using a solution delivery system; and (3) converting the precursor solution into a solid material by a pyrolysis reaction. The solution delivery system is used to drive the solution from a reservoir to a liquid injection nozzle that generates droplets with a size and velocity sufficient for their penetration into the core of a flame. The liquid flow rate and injection are controllable. Delivery of the solution typically comprises spraying of the solution into a chamber, onto the target substrate, or into a flame directed at the substrate. The substrate may be optionally heated. The resultant films may be optionally heat treated with an annealing procedure.

The precursor solution is formed from at least one precursor salt dissolved in a solvent or a combination of solvents. Exemplary salts include, but are not limited to, carboxylate salts, acetate salts, nitrate salts, chloride salts, alkoxide salts, butoxide salts and the like, and combinations comprising one or more of the foregoing salts; with alkali metals, alkaline earth metals, transition metals, rare earth metals, and the like, and combinations comprising one or more of the foregoing metals, as well as combinations of the foregoing salts and metals. Precursors may also be in the form of inorganic silanes such as, for example, tetraethoxysilane (TEOS), tetramethoxysilane (TMOS), and the like, and combinations comprising one or more of the foregoing silanes. Exemplary solvents in which the salts may be dissolved include, but are not limited to, water, alcohols, acetone, methyl ethyl ketone, and combinations comprising one or more of the foregoing solvents. The reagents are weighed according to the desired stoichiometry of the final compound and then added and mixed into a liquid medium. The precursor solution may be heated and stirred to dissolve the solid components and to homogenize the solution.

Coating may be conveniently accomplished using an aqueous solution reaction of appropriate precursors. An apparatus suitable to produce the nanostructured composite powders includes a reaction vessel equipped with a pH meter, temperature controller, hot plates and/or spray drier. Suitable process steps for the synthesis of nanostructured composites include precursor preparation; precomposite fabrication; nanostructured composite formation; and surface passivation.

In one embodiment of the solution precursor plasma spray, plasma spray may be accomplished in a manner to produce a particular microstructure of the thick film. The nanostructured material may be highly dense, for example greater than about 95% of the theoretical density.

The solution plasma spray method employed to produce the above-described microstructure comprises injecting precursor solution droplets into a thermal spray flame, wherein a first portion of the precursor solution droplets are injected into a hot zone of the flame, and a second portion of the precursor solution droplets are injected into a cool zone of the flame; fragmenting the droplets of the first portion to form reduced size droplets, and pyrolizing the reduced size droplets to form pyrolized particles in the hot zone; at least partially melting the pyrolized particles in the hot zone; depositing the at least partially melted pyrolized particles on the substrate; fragmenting at least part of the second portion of precursor solution droplets to form smaller droplets and forming non-liquid material from the smaller droplets; and depositing the non-liquid material on the substrate. The substrate may be optionally preheated and/or maintained at a desired temperature during deposition. As readily understood by one of ordinary skill in the art, the terms first portion and second portion do not imply a sequential order but are merely used to differentiate the two portions.

Prior to coating the thick film of the nanostructured material onto the particular structural member, a layer of the surface can be optionally oxidized. Especially, when the structural member is metallic, this oxidized layer can serve as a corrosion barrier to prevent the metallic structural member from undergoing corrosion and releasing metallic ions into the bloodstream. The oxidation can comprise preheating, electrolytic anodizing, passivating in a nitric acid bath, or the like.

Furthermore, after coating the thick film of the nanostructured material onto the structural member, and prior to characterization and/or implementation of the implant device, the thick film can optionally be further processed, e.g., abraded, ground and/or polished to adjust a coefficient of friction and/or surface roughness, plasma treated, sterilized, and the like. Additional layers can be added to provide additional functionality or desired characteristics to the thick coating. However, in one specific embodiment, the coated structural member is used as-is, that is, without grinding or further processing. In still another specific embodiment, the as-deposited thick film is abraded or polished as desired, but not further processed, e.g., not hydrated in order to enhance bonding between the coating and the substrate, not subjected to further coating, not consolidated, or the like. In such embodiments, the elimination of additional processing steps results in more economical manufacture of the medical devices.

The thickness of the deposited thick films is generally greater than or equal to about 25 micrometers (µm). In one embodiment, the average thickness of the thick film coating is greater than or equal to about 50 µm. In another embodiment, the average thickness of the thick film is greater than or equal to about 100 µm. In yet another embodiment, the thick film is less than or equal to about 3 millimeters (mm). It is postulated that thicker coatings (in particular greater than or equal to about 50 µm, and even more preferably greater than or equal to about 100 µm) advantageously provides greater protection to the support member material from scratching and wear effects, and/or less grain pull out (i.e., particulate debris) during interaction between surfaces. This can result in implants with service lifetimes that can be significantly prolonged. For example, a coating having an average thickness greater than or equal to about 50 µm is expected to last longer than a coating having an average thickness greater than or equal to about 25 µm. In turn, a coating having an average thickness greater than or equal to about 100 µm is expected to last longer than a coating having an average thickness greater than or equal to about 50 µm. In a clinical setting, a practitioner would accordingly prefer use of a medical device having a coating with a thickness greater than or equal to about 50 µm over a medical device having a coating with a thickness greater than or equal to about 25 µm.

The coating of the nanostructured material generally has a density greater than or equal to about 80 % of the theoretical density. In one embodiment, the density of the coating is greater than or equal to about 85 % of the theoretical density. In another embodiment, the density of the coating is greater than or equal to about 90 % of the theoretical density. In yet another embodiment, the density of the coating is greater than or equal to about 95 % of the theoretical density. Within the nanostructured material, the existing pores generally have an average longest dimension less than or equal to about 50 µm. In one embodiment, the average longest dimension of the pores within the nanostructured material is less than or equal to about 10 µm. In another embodiment, the average longest dimension of pores within the nanostructured material is less than or equal to about 1 µm.

The coating of the nanostructured material generally has a cross-sectional hardness (i.e., Vickers Hardness) greater than or equal to about 350 kilograms per square millimeter (kg/mm²). In one embodiment, the hardness of the coating is greater than or equal to about 500 kg/mm². In another embodiment, the hardness of the coating is greater than or equal to about 750 kg/mm². In yet another embodiment, the hardness of the coating is greater than or equal to about 1000 kg/mm². The hardness of the coating can be up to about 8,000 kg/mm².

The medical devices as described above can be used in accordance with their general purpose as is known to one of ordinary skill in the art. For example, an artificial implant is surgically implanted into the patient (animal or human).

The disclosure is further illustrated by the following non-limiting examples.

In these examples, characterization of products was carried out using several techniques. The phase of each product was identified by powder X-ray diffraction (XRD). Surface and cross-section microstructures were evaluated by scanning electron microscopy (SEM) using a JEOL model JSM-840A electron microscope. The average surface roughness of ground coatings was evaluated by a Mitutoyo SJ-201P Surface Roughness Tester, which is accurate to 0.01 micrometers. The coating crystal structure and grain size were observed by transmission electron microscopy (TEM). Cross-sectional hardness of coatings was measured using a Vicker's Test Model M-400-G2 at 300 gram (g), 1 kilogram (kg) and 2kg loads. Coating toughness was determined by the Palmqvist technique from crack lengths made by hardness indentations according to the formula of K_{IC}=0.016 (E/H)^{1/2}(P/C^{3/2}), wherein E is Young's modulus, H is the cross-sectional hardness, P is the load, and C is one-half of the crack distance.

The coefficient of friction for the coatings was estimated from wear data, per ASTM standard G99-95A, entitled "Standard Test Method for Wear Testing with a Pin-on-Disc Apparatus." The pin-on-disc machine was equipped with digital multimeters with 0.001 millivolts (mV) accuracy. The digital signals were input to a computer to record the data. The zero shift and other system errors were deduced according to the formula: V_{friction}=V_{turning}-V_{baseline}, wherein V_{friction} is the load cell generated voltage of the friction force, V_{turning} is the total voltage reading of the load cell when the system is running (i.e., the pin (specimen) is subjected to a weight imposed normal for contacting the disc) with the disc rotating clockwise, and V_{baseline} is the load cell voltage reading when the disc stops and the weight is removed. V_{baseline} is the bottom-line of the system without any frictional force, which reflects the system errors. The pins and discs used were ceramic coated 304 stainless steel alloys with flat surfaces and diameters of 3.81 and 31.75 mm, respectively. The lubricant was a 33 volume percent (vol %) solution of calf serum (MP Biomedicals Inc.) in deionized water. The test was run at room temperature under a 40 newton (N) load with a disc rotation of 240 revolutions per minute (RPM) in the lubricant.

The sliding wear rate of the ceramic coating was tested based on ASTM G99-95A, entitled "Standard Test Method for Wear Testing with a Pin-on-Disc Apparatus". The low contact pressure 3.51 MPa and travel speed 111.6 mm/s are similar to 3.55 MPa and twice that of 50.8mm/s for ASTM F 732-98, "Pin-on-Flat Wear Test for Polymeric Materials Used in Total Joint Processes Which Experience Linear Reciprocating Wear Motion." The pin was flat surfaced with diameter of 3.81 mm, disc turning speed of 240 RPM, track radius 0.35 inches (8.89 mm), and normal force 40 Newtons (N) at room temperature in 33 vol % calves serum. The ring-on-disc test was used for wear evaluations at high pressures, ranging from 12-16 MPa according to ISO 6474, "Implants for Surgery-Ceramic Materials Based on High Purity Alumina." The wear rate was calculated by weight-loss of the coating and the coating density for pin-on-disc test. The wear volume for ring-on-disc test was calculated by measuring the wear track depth and width using surface profilometer. An ultra high electronic balance was used to determine the weight loss with an accuracy within 10 micrograms. Analyses were guided by using ASTM F1714-97, "Guide for gravimetric wear assessment of prosthetic hip design in simulator devices." In order to eliminate the error of vapor condensation on samples, samples were vacuum dried at room temperature and sealed in dry N₂. At least four weighing data was recorded for each data. The weighing procedure was conducted in a dry N₂ environment. The testing room was environmentally controlled using an air conditioner combined with a dehumidifier at relative humidity 35%.

Tensile bond strengths were measured according to ASTM Standard C633-79 (re-approved in 1999), entitled "Test Method for Adhesion or Cohesive Strength of Flame-Sprayed Coatings." Shear bond strengths were measured according to ASTM Standard F 1044-99, entitled "Shear Testing of Calcium Phosphate Coatings and Metallic Coatings." The instrument was a standard tensile strength tester, model DH-10, made by United Calibration Corporation, CA. The uncoated side of each coupon was sand blasted using 30 # alumina granules before each bond strength test. The glue used for these tests was a FM 1000 glue film with a maximum tensile strength of 12,000 psi. Six samples were tested to obtain an average bond strength. Metallic rugs and coupons were fixed in a special holder, which maintained the applied load normal to the coating direction. A constant load was applied between the coating and the opposition device used to test the coating, using a calibrated high-temperature spring. The resultant stress was 0.138 megapascals (MPa), or about 20 psi. The bond strength test was performed with a constant crosshead speed of 0.12 centimeters per minute (cm/min). The fracture load and fracture surface were recorded.

### EXAMPLE 1: Thick chromia coated Ti₆Al₄V femoral head

The chromia feedstock composition contained 5 wt% SiO₂ and 3 wt% TiO₂, with the balance being Cr₂O₃. The average particle size of the raw powder materials was about 50 nm for Cr₂O₃ and SiO₂, and about 30 nm for TiO₂. A slurry containing the feedstock composition was first prepared with a powder to water ratio of 1:2. The feedstock composition was spray dried using a 16-foot industrial spray dryer with an inlet temperature of 446 °F, an outlet temperature of 105 °C, and a rotary speed of 25,000 RPM. The spray dried feedstock composition was partially sintered by heating at a temperature below 1000 °C in air, followed by heating at about 1000 °C to 1800 °C in hydrogen to agglomerate the nanoparticles into micrometer sized particles for thermal spraying. The feedstock included individual grains of about 20 - 50 nm agglomerated into about 5 - 100 micrometer sprayable agglomerates.

The feedstock was plasma thermal sprayed using a Metco 9MB plasma spray system equipped with GM-Fanuc 6-axis robot, a 2-axis specimen stage and a control console. To avoid potential heat damage in the Ti₆Al₄V alloy substrate (femoral head) and coating microcracking, a specific air-cooling jet system was used to decrease the substrate temperature during spray processing. The metallic substrate parts were sandblasted using 30 grit alumina granules. After sandblasting, a pre-oxidization of the substrate's metallic surface was carried out by heating with the plasma flame, electric anodizing, or soaking in nitric acid. The surface roughness of the sandblasted substrate was monitored using a Mitutoyo SJ201P surface profilometer. The optimized parameters for plasma thermal spray were: H₂/Argon plasma gas, 75 pounds per square inch (psi) gas pressure, 50 standard cubic feet per hour (SCFH) gas flow, 10 SCFH swirl gas flow, 600 amp arc current, 70 volt arc voltage, 310 nozzle type, 5 pounds per hour (lbs/hour) spray rate, 2.5 inch spray distance, PSA air jets, 35psi air pressure, 3.5 inch intersection, type11 power port, 450 surface feet per minute (SFPM) part rotation, 30 SCFH carrier gas flow, and 200 micrometer coating thickness. The coating thickness was determined to within an error of 0.1 µm by a Check-Line® 2000 coating thickness analyzer.

The ceramic coated femoral head was ground and polished using CNC grinding and polishing machines. For grinding, a Buehler Lapping oil or similar coolant was used. The grinding steps were: 1) grind using a 400 grit diamond wheel, 2) lap using a 6 micrometer diamond compound at 250 SFPM (1.27 meters per second (m/s)) for 15 minutes, 3) lap using a 1 micrometer diamond compound at 250 SFPM (1.27 m/s) for 10 minutes, and 4) lap using a 0.5 micrometer diamond compound at 400 SFPM (2.03 m/s) for 5 minutes. After grinding, a fine polishing step was carried out to obtain an average surface roughness (Ra) of about 0.01-0.02 micrometers in accordance with FDA standards.

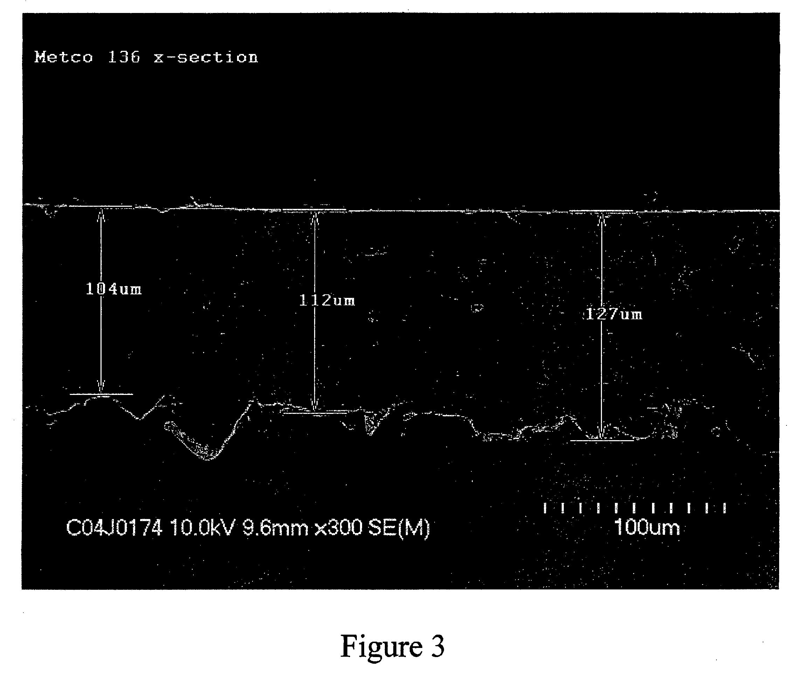

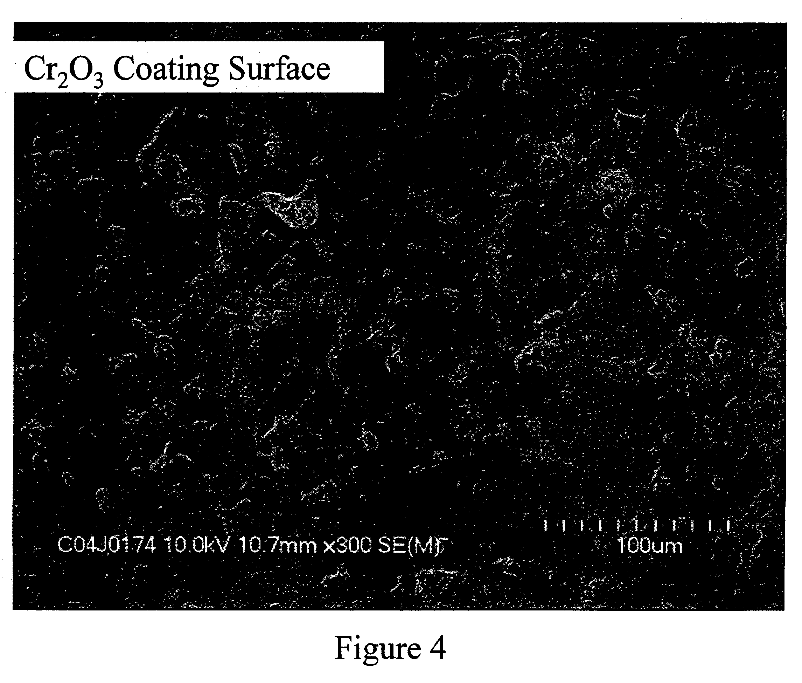

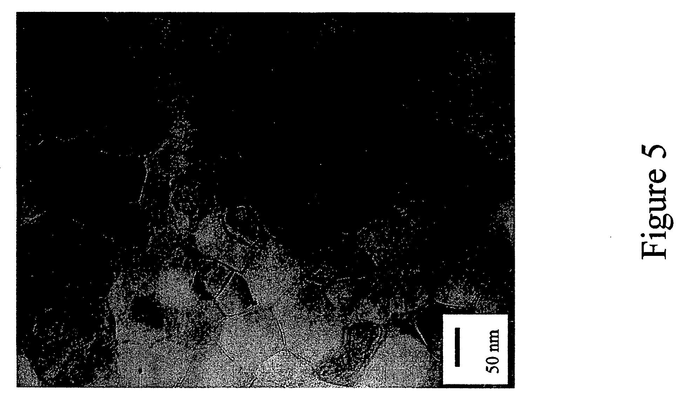

The thickness and microstructure of the coating was observed by scanning electron microscopy and transmission microscopy. Figure 3 illustrates that the coating thickness was about 100 micrometers after a final finishing step. The top surface had 5% volume percent pores for a lubricant reservoir as seen in Figure 4. The grain size of the thick Cr₂O₃ coatings is about 50 nm as evidenced in Figure 5. Other compositions (TiO₂ and SiO₂) of the coatings were amorphous, located at grain boundaries, and were difficult to reveal in the TEM bright field micrographs. However, the amorphous phase has been identified via selected area electron diffraction and dark field contrast analyses. PXRD revealed only a rhombohedral Cr₂O₃ phase.

The coatings exhibited greater than 95% density, tensile bond strengths above 40 Mpa, and shear bond strengths of 100 MPa. The coating bonded the substrate so well that there was no separation even after intentionally grinding the coating through to the metal substrate surfaces. The Cr₂O₃ coatings exhibited hardnesses of 1250 kg/mm² and fracture toughnesses of 13.3 MPa/m^{1/2}. The coefficient of friction was f=0.025 when the wear stayed in a stable range where the coating surface roughness was Ra=0.03-0.05 µm. Therefore, nanostructured Cr₂O₃ is a good candidate for use in orthopedic implants.

### EXAMPLE 2: (Comparative)

The chromia coated Ti₆Al₄V femoral head prepared according to Example 1 was compared to a commercial Co-Cr-Mo alloy on UHMWPE knee joint. As shown in Table 1, the improvements over the commercial product are significant.

**Table 1. Comparison of results for knee joints**

| Properties | Commercially Available Knee Joint | | Thick Film Coated Joints | |
|---|---|---|---|---|
| | Metal-on-UHMWPE | Ceramic coated metal-on-polymer | Ceramic coating | Estimated Improvements |
| Grain size | 10-30 µm (Metal) | 1-5 µm (Ceramic) | 50 nm (Ceramic) | ~100 times |
| Hardness kg/mm² | 320 (Metal) 5-13 (UHMWPE) | 1200 (Ceramic) 5-13 (UHMWPE) | 1250 (Ceramic) 1250 (Ceramic) | 100 times |
| Friction Coeff. | 0.02-0.03 | 0.02-0.03 | 0.025 | same |
| Wear Factor Linear Rate | 5×10⁻⁷ mm³/NM 0.17 mm/year | 2-7×10⁻⁸ mm³/NM 0.13 mm/year | 3.8×0⁻⁹ mm³/NM 0.00067 mm/year | 10-100 times -200 times |
| Longevity | 10-12 years | 15 years (estimated) | 15-25 years (estimated) | 5-10 years |

When converting the volume wear rate to a linear wear rate, the Cr₂O₃ coating-on-Cr₂O₃ coating had a linear wear rate of 0.67µm/year, which was about 200 times lower than the 170 µm/year for CoCrMo-on-UHMWPE knees and 130 µm/year for advanced ZrO₂-on-UHMWPE knees. The wear rate was among the best commercial bulk Al₂O₃-on-Al₂O₃ (Biolox®-forte) and bulk ZrO₂-on-ZrO₂ (Zilox®-forte). There is no comparison data for ring-on-disc for CoCrMo-on-UMWPE because the method was only designed for ceramic-on-ceramic wear, based on ISO 6474.

While the disclosure has been described with reference to exemplary embodiments, it will be understood by those skilled in the art that various changes may be made and equivalents may be substituted for elements thereof without departing from the scope of the disclosure. In addition, many modifications may be made to adapt a particular situation or material to the teachings of the disclosure without departing from the essential scope thereof. Therefore, it is intended that the disclosure not be limited to the particular embodiment disclosed as the best mode contemplated for carrying out this disclosure, but that the disclosure will include all embodiments falling within the scope of the appended claims.

## Claims

1. A medical device, comprising a structural member having a surface wherein at least a portion of the surface is coated with a nanostructured material to a thickness of at least about 25 micrometers, and wherein the nanostructured material comprises a ceramic, ceramic composite, ceramic metal composite, or a combination comprising at least one of the foregoing.

2. The medical device of Claim 1 wherein the medical device is an artificial implant.

3. The medical device of Claim 2, wherein the artificial implant comprises at least a portion of an articulating joint or a flexible hinge joint.

4. The medical device of Claim 3, wherein the articulating joint is used in partial or full replacement of a knee, hip, elbow, ankle, spine, shoulder, or wrist joint.

5. The medical device of Claim 3, wherein the flexible hinge joint is used in partial or full replacement of a silastic or metacarpal-phalangeal joint.

6. The medical device of any one of Claims 1 to 5, wherein the structural member is formed from an alloy, ceramic, or polymer.

7. The medical device of Claim 6, wherein the alloy structural member is a stainless steel alloy, titanium alloy, zirconium alloy, cobalt-chromium-molybdenum alloy, or a combination comprising at least one of the foregoing alloys.

8. The medical device of Claim 6, wherein the ceramic structural member is alumina, zirconia, titania, chromia, or a combination comprising at least one of the foregoing ceramics.

9. The medical device of Claim 6, wherein the polymer structural member is an ultra high molecular weight polyethylene.

10. The medical device of Claim 6 or 9, wherein the polymer structural member is reinforced with fibers, particulates, or a combination comprising at least one of the foregoing.

11. The medical device of any one of Claims 1 to 10, wherein the surface is a load bearing surface.

12. The medical device of Claim 6 or 7, further comprising an oxidized layer disposed between the surface of the alloy structural member and the nanostructured material.

13. The medical device of any one of Claims 1 to 12, wherein the nanostructured ceramic material is a hard phase metal oxide, metal carbide, diamond, metal nitride, metal boride, or a combination comprising at least one of the foregoing.

14. The medical device of any one of Claims 1 to 13, wherein the nanostructured ceramic composite material comprises at least 51 volume percent, based on a total volume of the ceramic composite, of a hard phase ceramic material and a binder phase, wherein the binder phase comprises SiO₂, CeO₂, Y₂O₃, TiO₂, or a combination comprising at least one of the foregoing.

15. The medical device of any one of Claims 1 to 14, wherein the nanostructured ceramic metal composite comprises WC/Co, TiC/Ni, TiC/Fe, Ni(Cr)/Cr₃C₂, WC/CoCr or a combination comprising at least one of the foregoing.

16. The medical device of Claim 15, further comprising TiC, VC, TaC, HfC, Cr, Ni, B, BN, or a combination comprising at least one of the foregoing.

17. The medical device of any one of Claims 1 to 16, wherein the nanostructured material has an average longest grain size dimension less than or equal to about 500 nanometers.

18. The medical device of any one of Claims 1 to 17, wherein the nanostructured material has an average longest grain size dimension less than or equal to about 100 nanometers.

19. The medical device of any one of Claims 1 to 18, wherein the thickness of the nanostructured material coating is less than or equal to about 3 millimeters.

20. A medical device, comprising a first structural member having a first surface and a second structural member having a second surface, wherein at least a portion of one or both of the first and second surfaces is coated with a nanostructured material to a thickness of at least about 25 micrometers.

21. The medical device of Claim 20, wherein one or both of the first and second surfaces is a load bearing surface.

22. The medical device of Claim 20 or 21, wherein the medical device is an articulating joint or a flexible hinge joint.

23. The medical device of Claim 22, wherein the articulating joint is used in partial or full replacement of a knee, hip, elbow, ankle, spine, shoulder, or wrist joint.

24. The medical device of Claim 22, wherein the flexible hinge joint is used in partial or full replacement of a silastic or metacarpal-phalangeal joint.

25. The medical device of any one of Claims 20 to 24, wherein one or both of the first and second structural members is formed from an alloy, ceramic, polymer, or cartilage.

26. The medical device of Claim 25, wherein the alloy is a stainless steel alloy, titanium alloy, zirconium alloy, cobalt-chromium-molybdenum alloy, or a combination comprising at least one of the foregoing alloys.

27. The medical device of Claim 25, wherein the ceramic is alumina, zirconia, titania, chromia, or a combination comprising at least one of the foregoing ceramics.

28. The medical device of Claim 25, wherein the polymer is an ultra high molecular weight polyethylene.

29. The medical device of Claim 25 or 28, wherein the polymer is reinforced with fibers, particulates, or a combination comprising at least one of the foregoing.

30. The medical device of any one of Claims 20 to 29, wherein the first and second surfaces are pivotably engageable.

31. The medical device of any one of Claims 20 to 30, wherein the nanostructured material is a ceramic, ceramic composite, ceramic metal composite, or a combination comprising at least one of the foregoing.

32. The medical device of Claim 31, wherein the ceramic is a hard phase metal oxide, metal carbide, diamond, metal nitride, metal boride, or a combination comprising at least one of the foregoing.

33. The medical device of Claim 31, wherein the ceramic composite comprises at least 51 volume percent, based on a total volume of the ceramic composite, of a hard phase ceramic material and a binder phase, wherein the binder phase comprises SiO₂ CeO₂, Y₂O₃, TiO₂, or a combination comprising at least one of the foregoing.

34. The medical device of Claim 31, wherein the ceramic metal composite comprises WC/Co, TiC/Ni, TiC/Fe, Ni(Cr)/Cr₃C₂, WC/CoCr or a combination comprising at least one of the foregoing.

35. The medical device of any one of Claims 20 to 34, wherein the nanostructured material has an average longest grain size dimension less than or equal to about 500 nanometers.

36. The medical device of any one of Claims 20 to 35, wherein the nanostructured material has an average longest grain size dimension less than or equal to about 100 nanometers.

37. The medical device of any one of Claims 20 to 36, wherein the thickness of the nanostructured material coating is less than or equal to about 3 millimeters.

38. A method, comprising surgically implanting a medical device comprising a structural member having a surface, wherein at least a portion of the surface is coated with a nanostructured material to a thickness of at least about 25 micrometers, and wherein the nanostructured material comprises a ceramic, ceramic composite, ceramic metal composite, or a combination comprising at least one of the foregoing.

39. The method of Claim 38, wherein the medical device comprises at least a portion of an articulating joint or a flexible hinge joint.

40. The method of Claim 39, wherein the articulating joint is used in partial or full replacement of a knee, hip, elbow, ankle, spine, shoulder, or wrist joint.

41. The method of Claim 39, wherein the flexible hinge joint is used in partial or full replacement of a silastic or metacarpal-phalangeal joint.

42. A method of making a medical device, comprising coating at least a portion of at least one surface of the medical device with a film of a nanostructured material having a thickness of at least about 25 micrometers.

43. The method of Claim 42, wherein the at least one surface is formed from an alloy comprising a stainless steel alloy, titanium alloy, zirconium alloy, cobalt-chromium-molybdenum alloy, or a combination comprising at least one of the foregoing alloys.

44. The method of Claim 43, further comprising oxidizing a layer of the alloy surface to provide a corrosion barrier effective to decrease any corrosion or release of metal ions into a bloodstream, prior to coating.

45. The method of Claim 44, wherein oxidizing comprises heating, anodizing, passivating, or a combination comprising at least one of the foregoing.

46. The method of any one of Claims 42 to 45, wherein coating comprises thermal spraying, chemical vapor deposition, physical vapor deposition, sputtering, ion plating, cathodic arc deposition, atomic layer epitaxy, molecular beam epitaxy, powder sintering, electrophoresis, electroplating, injection molding, or a combination comprising at least one of the foregoing.

47. The method of any one of Claims 42 to 46, further comprising annealing the film.

48. The method of any one of Claims 42 to 47, further comprising grinding the film.

49. The method of any one of Claims 42 to 48, further comprising polishing the film.

50. The method of any one of Claims 42 to 49, wherein the nanostructured material has an average longest grain size dimension less than or equal to about 500 nanometers.

51. The method of any one of Claims 42 to 50, wherein the nanostructured material comprises a ceramic, ceramic composite, ceramic metal composite, alloy, or a combination comprising at least one of the foregoing.

52. The method of any one of Claims 42 to 51, wherein the film has a thickness less than or equal to about 3 millimeters.
